# EUROPÄISCHE PATENTSCHRIFT

(11) **EP 2 112 909 B1**
(45) Veröffentlichungstag und Bekanntmachung des Hinweises auf die Patenterteilung: **11.03.2015**
(21) Anmeldenummer: 08773424.0
(22) Anmeldetag: 13.06.2008
(51) Int. Cl.: A61B 18/14, A61B 17/285, A61B 17/3201, A61B 17/29, A61B 17/3211

(54) **CHIRURGISCHES INSTRUMENT**
SURGICAL INSTRUMENT
INSTRUMENT CHIRURGICAL

(30) Priorität: 29.06.2007 DE 202007009165 U
(43) Veröffentlichungstag der Anmeldung: 04.11.2009
(73) Patentinhaber: KLS Martin GmbH + Co. KG, 79224 Umkirch (DE)
(72) Erfinder: FRITZSCH, Gernod, 78532 Tuttlingen (DE)
(74) Vertreter: Maucher Börjes Jenkins Patent- und Rechtsanwälte
(86) Internationale Anmeldenummer: PCT/EP2008/004748
(87) Internationale Veröffentlichungsnummer: WO 2009/003575

(56) Entgegenhaltungen:
- EP-A- 1 810 625
- WO-A-02/080794
- WO-A-2008/008457
- US-A1- 2003 216 733

## Beschreibung

Die Erfindung betrifft ein chirurgisches Instrument mit zwei an einem Ende eines Schafts oder eines Griffteils befindlichen Klemmbacken, und mit einer einen durchgehenden Lagerbolzen aufweisenden Lagerung, an welcher zumindest eine der Klemmbacken zwischen einer Offen- und einer Schließstellung schwenkbar gelagert ist, und mit einer im wesentlichen parallel zu der Längsachse des Instruments in einen zwischen den Klemmbacken befindlichen Zwischenraum verschiebbaren Trenneinrichtung.

Mit solchen chirurgischen Instrumenten werden in effektiver Weise mehrere Arbeitsschritte in der chirurgischen Behandlung von biologischem Gewebe mit nur einem Instrument durchgeführt, nämlich die Versiegelung des Gewebes mittels bipolarer Koagulation sowie eine mechanische Trennung von Gewebeabschnitten. Mittels der Backen des Instruments wird hierbei zunächst ein Gewebebereich erfasst und gehalten, zusammengedrückt und dann durch im Bereich der Backen angeordnete, gegeneinander isolierte Elektroden koaguliert. Hierbei wird durch den zwischen den Elektroden durch das Gewebe fließenden Strom selbiges erwärmt und durch den gleichzeitig auf den Koagulationsbereich ausgeübten mechanischen Druck die Blutgefäße dort versiegelt. Nach der Versiegelung wird ein Schneidinstrument durch das zwischen den Elektroden befindliche Gewebe geschoben und dieses durchtrennt.

Ein entsprechendes, zur Durchführung eines eben beschriebenen Trennvorgangs geeignetes chirurgisches Instrument, ist etwa aus der EP 1 372 507 bekannt. Es handelt sich dabei um ein Koagulationsinstrument, bei welchem beide Klemmbacken um einen gemeinsamen durchgehenden Lagerbolzen schwenkbar am Ende eines Rohrschafts angeordnet sind. Als Trenneinrichtung dient eine Klinge, die im Bereich des Lagerbolzens ein Langloch aufweist, um die relative Verschiebung in den Zwischenraum zwischen den beiden Klemmbacken zu ermöglichen.

Die Existenz des Langlochs in der Klinge führt zwangsläufig dazu, dass die Klinge der Trenneinrichtung nicht ausgewechselt werden kann und das Instrument daher allenfalls als Einmalinstrument geeignet ist. Da jedoch die Klinge der Trenneinrichtung im Laufe von Schneidvorgängen stumpf werden kann, ist es zweckmäßig, sie für eine Mehrfachbenutzung auswechselbar auszulegen. Überdies ist die Klinge dann auch einfacher zu reinigen.

Aus der DE 10 2004 040 959 A1 ist ein vergleichbares Instrument bekannt, bei welchem eine der beiden Klemmbacken schwenkbar und die andere feststehend ist. Die Trenneinrichtung dieses Instruments wird im Bereich des Lagerbolzens mit einer schmalen Schubstange bewegt, die die gegenüber der Schubstange breitere Klinge verschiebt. Der Lagerbolzen muss daher unterbrochen, also zweiteilig beidseitig der Schubstange oder über oder unter dieser angeordnet sein, was hinsichtlich der Festigkeit der Schwenklagerung ungünstig ist und die Montage erschwert. Auch ein Zurückziehen der gegenüber der Schubstange erheblich breiteren Klinge aus dem Arbeitsbereich zum Zwecke des Reinigens oder der Auswechslung gestaltet sich aufgrund dieser geometrischen Gegebenheiten schwierig.

Aus WO 2006/008457 A2 ist eine Lösung mit einem vergleichbaren Instrument bekannt, bei welchem die Trenneinrichtung ebenfalls eine sie erforderlichenfalls bewegende Schubstange in ihrer Abmessung erheblich übertrifft und nur die Schubstange durch eine Durchtrittsöffnung des Lagerbolzens verschiebbar ist, nicht aber die Trenneinrichtung, so dass sie nicht für eine Auswechselung relativ zu dem Lagerbolzen aus diesem zurückgezogen werden kann.

Es besteht daher die Aufgabe, ein Instrument der eingangs genannten Art zu schaffen, welches einfach aufgebaut eine stabile Schwenklagerung bietet und dennoch die Trenneinrichtung in einfacher Weise zum einen in den Bereich zwischen den Klemmbacken verschiebbar und zum anderen zu Reinigungs- oder Auswechselzwecken auch leicht in proximaler Richtung zurückziehbar ist.

Diese Aufgabe wird mit den Mitteln und Merkmalen des Patentanspruchs 1 gelöst.

Vor allem ist bei einem eingangs definierten chirurgischen Instrument vorgesehen, dass der durchgehende Lagerbolzen eine sich entlang der Bewegungsrichtung der Trenneinrichtung erstreckende Durchtrittsöffnung aufweist, durch welche die Trenneinrichtung durchsteckbar ist, und dass die größte Erstreckung der Durchtrittsöffnung quer zur Bewegungsrichtung der Trenneinrichtung wenigstens der Abmessung der Trenneinrichtung in dieser Richtung entspricht.

Somit kann an der Lagerung der Klemmbacken des chirurgischen Instruments ein durchgehender, eine Durchtrittsöffnung aufweisender Lagerbolzen in Art eines Durchbruchs vorgesehen sein, der der Lagerung ein hohes Maß an Stabilität verleiht und gleichzeitig dennoch ein Zurückziehen der Trenneinrichtung für ein Auswechseln ermöglicht.

Die betreffende Trenneinrichtung des erfindungsgemäßen chirurgischen Instruments lässt sich in besonders einfacher und vorteilhafter Weise realisieren, wenn sie als Trenneinrichtung mit einer Klinge ausgeführt ist. Daneben sind aber auch andere Trenneinrichtungen, beispielsweise in Form einer Stanze oder eines anderen geeigneten Trennmittels, zum Beispiel einer elektrischen Hochfrequenz-Schneide, denkbar.

Eine vielseitige, an verschiedene Anforderungen anpassbare und darüber hinaus einfach herstellbare Ausbildung der Schwenklagerung kann durch eine Weiterbildung des chirurgischen Instruments erreicht werden, bei der der Lagerbolzen als rotationssymmetrischer, insbesondere bereichsweise zylindrischer Körper mit gegebenenfalls sich änderndem Querschnitt ausgebildet ist, so dass unterschiedliche chirurgische Instrumente mit dem betreffenden Lagerbolzen versehen sein können.

Eine stabile, den auf den Lagerbolzenden wirkenden Kräften gut standhaltende Schwenklagerung kann bei einer zweckmäßigen Weiterbildung des chirurgischen Instruments dadurch gewährleistet werden, dass der Lagerbolzen seinen größten Querschnitt quer zur Bewegungsrichtung der Trenneinrichtung in einem Abschnitt aufweist, in welchem die Durchtrittsöffnung angeordnet ist.

Erfindungsgemäß ist die Durchtrittsöffnung im wesentlichen quer zur Längsachse der Lagerung schlitzartig verlaufend vorgesehen.

Hierbei verläuft dann die größere Ausdehnung des betreffenden Schlitzes quer zur Längserstreckung des Lagerbolzens. Somit kann die Trenneinrichtung hochkant durch diese schlitzartige Durchtrittsöffnung hindurchbewegt werden. Aufgrund seiner Form weist die schlitzförmige Durchtrittsöffnung darüber hinaus auch eine Führungsfunktion auf, da sie der Trenneinrichtung nicht nur den notwendigen Platz gewährt, sondern diese an deren Seitenwänden bzw. -rändern gleichzeitig auch geführt ist. Hierzu kann die Trenneinrichtung beispielsweise an den Innenwandungen der Durchtrittsöffnung entlang gleiten.

Die Stabilität der Schwenklagerung sowie eine sichere Führung der Trenneinrichtung können in besonderem Maße bei einer Weiterbildung des chirurgischen Instruments gewährleistet werden, bei der die an der Lagerung befindliche Durchtrittsöffnung allseitig geschlossen ist, deren Seitenwände also allseitig eine Stützfunktion übernehmen können, während die Trennenrichtung allseitig von Innenwandungen der Durchtrittsöffnung umfasst ist.

Bei einer anderen, gleichwohl ebenfalls stabilen Ausführungsform, die eine Entnahme der Trenneinrichtung gegebenenfalls erleichtert, kann demgegenüber die an der Lagerung befindliche Durchtrittsöffnung mit einer insbesondere über die gesamte Längserstreckung der Durchtrittsöffnung unterbrochenen Umfangswand einseitig offen ausgebildet sein. Die Innenwandung eines derartigen Lagerbolzens kann dann beispielsweise einen U-förmigen Querschnitt aufweisen.

Eine sichere Schnittführung einer Bedienperson des chirurgischen Instruments wird optimal durch eine exakt mittige Führung der Trenneinrichtung entlang der Mittelebene der Klemmbacken unterstützt, weswegen eine sinnvolle Ausführung des Instruments darin besteht, die Durchtrittsöffnung derart in der Mitte des Lagerbolzens der Lagerung anzuordnen, dass die Mitte der Durchtrittsöffnung eine Symmetrieebene hinsichtlich der sich zu beiden Seiten ersteckenden Teile des Lagerbolzens bildet.

Möglichst platzsparend kann der Lagerbolzen an dem beispielsweise durch ein Rohrschaftinstrument gebildeten erfindungsgemäßen chirurgischen Instrument untergebracht werden, wenn ein mit der Durchtrittsöffnung versehener Mittelabschnitt des Lagerbolzens einen größeren Querschnitt als dessen Lagerenden aufweist und diese Lagerenden dann wiederum einfach in für sie vorgesehene Öffnungen eingreifen können.

Zur Erhöhung des Stabilität des Lagerbolzens und auch seines durchbrochenen Bereichs kann es zweckmäßig sein, an dem an dem Lagerbolzen zwischen dem Mittelabschnitt und dem jeweiligen Lagerende einen Zwischenabschnitt vorzusehen, dessen Durchmesser zwischen dem des Mittelabschnitts und dem des Lagerendes liegt.

Besonders bevorzugt ist hierbei eine Ausbildung des Lagerbolzens mit einem Zwischenabschnitt, dessen radiale Ausdehnung gleich groß oder größer als die größte radiale Ausdehnung des Durchtrittskanals ist, so dass die Seitenwandungen der Durchtrittsöffnung in Richtung zu den Lagerenden des Lagerbolzens hin besser abgestützt werden können.

Sowohl vor als auch während eines Operationsvorgangs mit dem erfindungsgemäßen chirurgischen Instrument kann ein Austausch der Trenneinrichtung notwendig sein, wobei dieser Austausch dadurch vereinfacht werden kann, dass die Ränder der Eintrittsöffnung und/oder der Austrittsöffnung der Durchtrittsöffnung zumindest bereichsweise abgeflacht sind, insbesondere gegenüberliegende Längsränder einer schlitzartigen Durchtrittöffnung angefast sind. Die abgeflachten Bereiche erleichtern hierbei das Führen der Trenneinrichtung in die betreffende Öffnung, auch wenn diese von dem betreffenden Ende der Trenneinrichtung nicht exakt getroffen wird.

Um den Lagerbolzen fest und sicher an einem Gehäuse, beispielsweise an dem Schaft eines Rohrschaftinstruments anordnen zu können, ist es zweckmäßig, an den Lagerenden des durchgehenden Lagerbolzens Bereiche zur Anlage eines Widerlagers zu sehen. Dies können etwa Bereiche sein, in denen die Zylinderform abgeflacht ist, also solche Bereiche, in denen die Rotationsymmetrie des Lagerbolzens gegebenenfalls gebrochen ist.

Da die erfindungsgemäße Anordnung des chirurgischen Instruments ein einfaches Auswechseln einer Klinge des Schneidinstruments vorteilhaft unterstützt, ist es weiter zweckmäßig, die Trenneinrichtung mit mindestens einer Betätigungseinrichtung und gegebenenfalls mit einer Halterung zu versehen, an welcher diese Klinge lösbar befestigt ist.

Bei Verwendung des chirurgischen Instruments als Koagulationsinstrument sind im Bereich der Klemmbacken verschiedene Abschnitte als Elektroden vorgesehen, die den für die Elektrokoagulation des Gewebes notwendigen Strom zur Verfügung stellen und welche in der Regel an den Innenflächen der Klemmbacken angeordnet sind. Damit zum einen die Klinge des chirurgischen Instruments über die vorgesehene bzw. gewünschte Schnittlänge an den Klemmbacken definiert in den zwischen den Klemmbacken befindlichen Zwischenraum geführt werden kann, zum anderen hierdurch keine leitende Verbindung zwischen den Klemmbacken hergestellt wird, ist es bei einer vorteilhaften Weiterbildung des chirurgischen Instruments vorgesehen, dass die Trenneinrichtung von dem proximalen bis in den Bereich des distalen Endes der Klemmbacken in einer in Verlängerung der Durchtrittsöffnung an den Klemmbacken angeordneten, mit dieser fluchtenden Führung, insbesondere in einer Nut, geführt ist.

Eine zweckmäßige Verwendung des erfindungsgemäßen chirurgischen Instruments stellt, wie bereits beschrieben, dessen Integration in ein Koagulationsinstrument, insbesondere ein Hochfrequenz-Koagulationsinstrument, dar.

Die Erfindung wird nachstehend anhand von Ausführungsbeispielen in der Zeichnung näher erläutert. Dabei zeigen in teilweise schematisierter Darstellung die
- Fig.1: eine perspektivische Seitenansicht eines ersten Ausführungsbeispiels eines chirurgischen Instruments als Rohrschaftinstrument, an dessen distalem Ende Klemmbacken mit einem Lagerbolzen angeordnet sind;
- Fig. 2: eine perspektivische Seitenansicht einer vergrößerten Darstellung des distaslen Endes des chirurgischen Instruments aus der Fig.1;
- Fig. 3: eine perspektivische Teilansicht der Darstellung aus der Fig.2;
- Fig. 4: eine geschnittene Seitenansicht des durchgehenden Lagerbolzens bei geschlossenen Klemmbacken der Ausführungsform des chirurgischen Instruments der Fig.1;
- Fig. 5: eine perspektivische Seitenansicht einer ersten Ausführung des durchgehenden Lagerbolzens;
- Fig. 6: eine perspektivische Seitenansicht einer weiteren Ausführung des durchgehenden Lagerbolzens;
- Fig. 7: eine Seitenansicht einer anderen Ausführungsform des chirurgischen Instruments mit nur einer beweglichen Klemmbacke;
- Fig. 8: eine geschnittene Seitenansicht des Instruments aus der Fig.7 entlang der Linie C-C;
- Fig. 9: eine perspektivische Ansicht des Instruments der Fign. 7 und 8;
- Fig. 10: eine perspektivische Seitenansicht einer weiteren Ausführungsform des chirurgischen Instruments;
- Fig. 11: eine perspektivische Seitenansicht einer Ausführung eines durchgehenden, einstückigen Lagerbolzens, wie er in der Fig. 10 verwendet wird;
- Fig. 12: eine ebene Aufsicht auf das distale Ende des Instruments aus der Fig.10; und die
- Fig. 13: eine geschnittene Seitenansicht des chirurgischen Instruments aus der Fig. 12 entlang der Linie B-B.

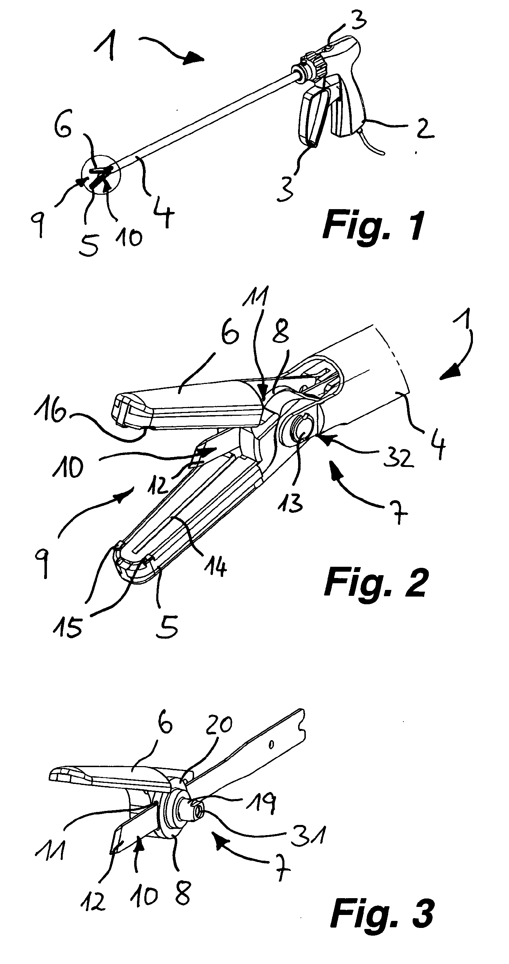

In der Fig.1 ist ein im Ganzen mit 1 bezeichnetes chirurgisches Instrument dargestellt, welches als Rohrschaftinstrument ausgebildet ist. Von einem dem Betrachter abgewandten, proximalen Ende des Instruments 1, an welchem sich ein Griffteil 2 befindet, erstreckt sich auf den Betrachter zu zunächst ein Schaft 4. An dessen dem Betrachter zugewandtem, distalen Ende sind zwei Klemmbacken 5, 6 sowie eine Lagerung 7 (vgl. Fig. 2) mit einem durchgehenden, also einteiligen Lagerbolzen 8 angeordnet, an welchem die beiden Klemmbacken 5, 6 zwischen einer Offenund einer Schließstellung schwenkbar gelagert sind. An dem Griffteil 2 des Instruments 1 befinden sich außerdem mehrere Betätigungseinrichtungen 3, mittels derer die unterschiedlichen Funktionen des chirurgischen Instruments 1 wie Halten, Koagulieren und Schneiden ausgelöst werden können. Darüber hinaus weist das chirurgische Instrument eine sich im wesentlichen parallel zu der Längsachse des Instruments 1 in einen zwischen den Klemmbacken 5, 6 befindlichen Zwischenraum 9 verschiebbare Trenneinrichtung 10 auf.

Der dem Betrachter zugewandte distale Bereich des chirurgischen Instruments 1 der Fig. 1 mit den Klemmbacken 5, 6 in Offenstellung ist genauer in der Fig. 2 zu erkennen, der zu entnehmen ist, dass der Lagerbolzen 8 der Lagerung 7 eine sich in Bewegungsrichtung der Trenneinrichtung 10 erstreckende Durchtrittsöffnung 11 aufweist, durch die die Trenneinrichtung 10 durchgesteckt ist, wobei die größte Erstreckung der Durchtrittsöffnung 11 quer zur Bewegungsrichtung der Trenneinrichtung 10 der Abmessung der Trenneinrichtung 10 in dieser Richtung entspricht. Die Trenneinrichtung 10 ragt hierbei in der Darstellung der Fig.2 die Durchtrittsöffnung 11 durchgreifend mit dem distalen Ende ihrer Klinge 12 in den zwischen den offenen Klemmbacken 5, 6 gebildeten Zwischenraum 9. An dem dem Betrachter zugewandten Ende der Klinge 12 ist als geschliffener Teil eine Schneide 17 angeordnet, die von ihrem der oberen Klemmbacke zugewandten Endbereich in Richtung des unteren Endbereichs leicht vorspringend verläuft. Die Klemmbacken 5, 6 sind an dem Lagerbolzen 8 der Lagerung 7 gegeneinander verschwenkbar gelagert, der wiederum mittels zweier Nieten 13, von denen nur der dem Betrachter zugewandte sichtbar ist, an dem Ende des Schafts 4 des Rohrschaftinstruments dadurch befestigt ist, dass dieser Niet 13 eine Schaft-öffnung 32 durchgreift. Die die Schwenkbewegung der Klemmbacken 5, 6 verursachenden Steuerelemente sind hierbei der besseren Übersichtlichkeit halber nicht näher dargestellt. Ebenfalls ist der Fig.2 zu entnehmen, dass die Trenneinrichtung 10, bzw. deren Klinge von dem proximalen bis in den Bereich des distalen Endes der Klemmbacken 5, 6 in einer in Verlängerung der Durchtrittsöffnung 11 an den Klemmbacken 5, 6 angeordneten, mit dieser fluchtenden Führung 14 in Art einer Nut geführt ist. Um zu verhindern, dass sich die mit nicht weiter dargestellten Elektroden versehenen, einander zugewandten Stirnflächen der Klemmbacken 5, 6 berühren, ist die eine Klemmbacke 5 in ihrem Randbereich mit zwei zapfenartig von der Stirnseite der Klemmbacke 5 abstehenden Abstandshaltern 15 versehen, die in komplementäre, an der Stirnseite der anderen Klemmbacke 6 vorgesehene Ausnehmungen 16 eingreifen. Die Höhe der Ausnehmungen 16 ist dabei geringer als diejenige der Abstandshalte, so dass auch in Schließstellung der Klemmbacken 5, 6 ein geringer Zwischenraum 9 zwischen diesen verbleibt.

In der Fig.3 ist der besseren Übersichtlichkeit halber die Darstellung der Fig.2 in einer Teilansicht gezeigt, in welcher die obere, an dem Lagerbolzen 8 angeordnete Klemmbacke 6 zu erkennen, sowie die Trenneinrichtung 10, die mit ihrer Klinge 12 die allseitig geschlossene Durchtrittöffnung 11 durchgreift. Ebenso ist hier zu erkennen, dass der Lagerbolzen 8 der Lagerung 7 als im wesentlichen rotationssymmetrischer Körper mit sich änderndem Querschnitt ausgebildet ist, da der Querschnitt des Lagerbolzens sich von seiner mit der Durchtrittsöffnung 11 versehenen Mitte, nämlich einem scheibenartigen Mittelabschnitt 20, zu seinen beiden Lagerenden 19 hin verjüngt. Die Trenneinrichtung 10 ist in ihrem rückwärtigen, dem Betrachter abgewandten Bereich mit einer lochartigen, nicht weiter bezeichneten, die Fläche der Trenneinrichtung durchmessenden Öffnung versehen, an welcher eine nicht weiter dargestellte, die Trenneinrichtung 10 vor- und zurückziehende Betätigungseinrichtung angreift. An der Stirnfläche des Lagerendes 19 ist der Lagerbolzens 8 mit einem Eingriff 31 versehen, in welchen ein Befestigungsmittel, welches vorher die Öffnung des Rohrschafts durchgreift, eingreifen kann, also eine Schraube, ein Niet 13 oder dergleichen.

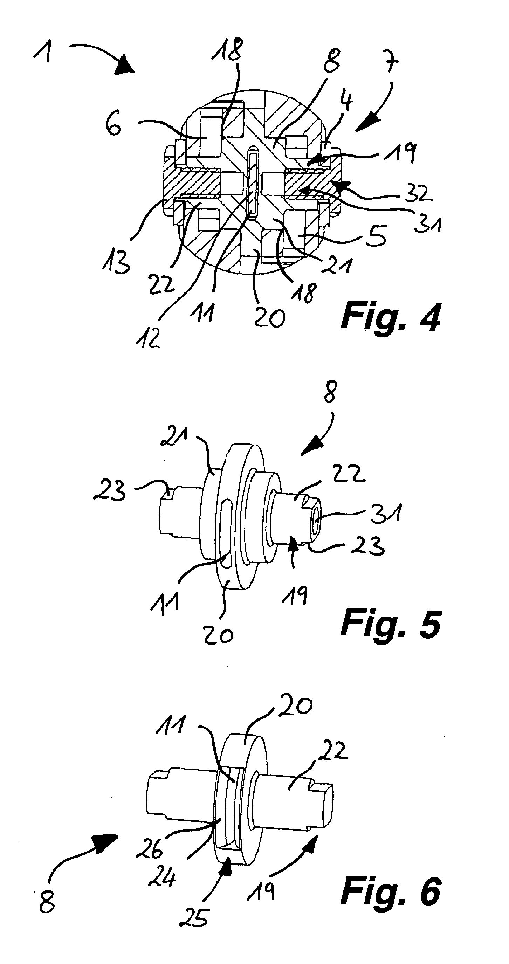

Die Fig.4 zeigt das chirurgische Instrument der vorhergehenden Figuren in einer im Bereich der Lagerung 7 geschnittenen Seitenansicht. Deutlich ist wiederum der Lagerbolzen 8 mit der Durchtrittsöffnung 11 zu erkennen, in welche die Trenneinrichtung 10 eingeführt erkennbar ist. Der Lagerbolzen 8 ist mittels zweier Nieten 13 an dem distalen Ende des Schaftes 4 des Rohrschaftinstruments festgelegt, auch andere geeignete Befestigungsmittel, bspw. in der Form von Gewindeschrauben wären an dieser Stelle verwendbar. Die Nieten 13, die einen leicht größeren Durchmesser als die kreisrunden Eingriffe 31 an den Stirnseiten der Lagerbolzen aufweisen, durchgreifen hierbei zunächst die Schaftöffnungen 32 des Schafts 4 und tauchen sodann eben in diese Eingriffe 31 ein. Von einem Mittelabschnitt 20 größter radialer Ausdehnung des Lagerbolzens 8, in welchem sich die Durchtrittsöffnung 11 befindet nimmt der Querschnitt des Lagerbolzens 8 in einem Zwischenabschnitt 21 auf eine mittlere radiale Ausdehnung ab, die immer noch größer als die radiale Ausdehnung der Durchtrittsöffnung 11 ist. An diesem scheibenförmigen Absatz, der eine radiale und eine axiale Fläche bildet, sind die Klemmbacken 5, 6 dadurch gelagert, dass der Absatz in Rücksprünge 18 der Klemmbacken 5, 6 eingreift. Zu den Lagerenden 19 hin nimmt der Querschnitt des Lagerbolzens 8 erneut ab, so dass abgeflachte Bereiche 23 (vgl. Fig.5) der Lagerenden 19 in Öffnungen an dem Ende des Schafts 4 eingreifen und der Lagerbolzen 8 dort festgelegt werden kann.

Diese Verhältnisse sind erneut auch der Fig.5 entnehmbar, in welcher der Lagerbolzen 8 des chirurgischen Instruments der Figuren 1 bis 4 nochmals einzeln dargestellt ist. In einem scheibenartigen Mittelabschnitt 20 des durchgehenden Lagerbolzens 8 befindet sich die Durchtrittsöffnung 11 für die nicht dargestellte Trenneinrichtung 10, wobei die Mitte der Durchtrittsöffnung 11 eine Symmetrieebene hinsichtlich der sich zu beiden Seiten erstreckenden Teile des Lagerbolzens 8 bildet. Zu beiden Seiten des Mittelabschnitts 20 schließt sich ein Zwischenabschnitt 21 reduzierten Querschnitts an, wobei dieser Querschnitt eine größere radiale Ausdehnung als diejenige der Durchtrittsöffnung 11 aufweist, so dass die Durchtrittsöffnung 11 seitlich gut abgestützt ist. An den Mittelabschnitt 21 schließen sich jeweils die Endabschnitte 22 an, die in die Lagerenden 19 münden. An den Lagerenden 19 des Lagerbolzens 8 befinden sich jeweils zwei von der zylindrischen Form ausgenommene, abgeflachte Bereiche 23 zur Anlage an Widerlagern.

In der Fig.6 ist eine andere Ausführung des Lagerbolzens gezeigt, bei welcher der Zwischenabschnitt 21 fehlt, also der mit der Durchtrittsöffnung 11 versehene Mittelabschnitt direkt in die Endabschnitte 22 mit den abgeflachten Bereichen 23 übergeht. Dafür ist die Durchtrittsöffnung 11 des Lagerbolzens 8 der Fig.6 an ihren gegenüberliegenden Längsrändern 24 der gezeigten Austrittsöffnung 25 und der auf der abgewandten Seite des Lagerbolzens befindlichen Eintrittsöffnung abgeflacht, die Längsränder sind über ihre gesamte Länge mit einer Fase 26 versehen.

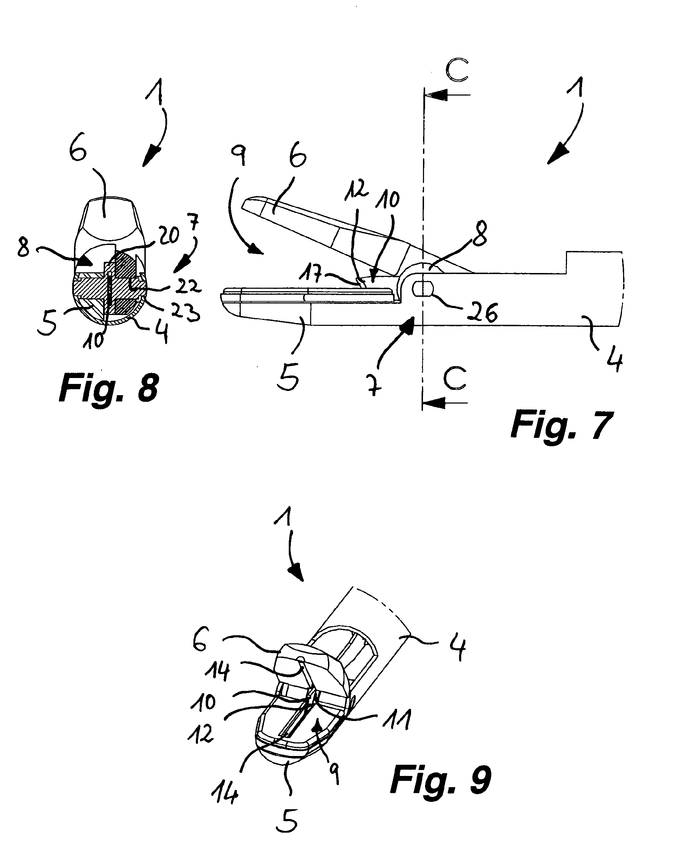

Die Fig.7 zeigt die Offenstellung einer anderen Ausführungsform des chirurgischen Instruments 1, welches zwar ebenfalls ein Rohrschaftinstrument ist, bei welchem jedoch nur die eine, für den Betrachter obere Klemmbacke 6 beweglich gegenüber der anderen, unteren Klemmbacke 5 ausgeführt ist, während letztere als Endbereich einen Teil des Schafts 4 bildet. Die obere Klemmbacke 6 ist dabei schwenkbeweglich an dem Lagerbolzen 8 gelagert, der eine Durchtrittsöffnung 11 aufweist, durch welche die Trenneinrichtung 10 durchgesteckt ist. Diese ragt in den in der Offenstellung der Fig.7 gut erkennbaren, zwischen den Klemmbacken 5, 6 befindlichen Zwischenraum 9. Anders als in der ersten Ausführungsform des chirurgischen Instruments 1 ragt hier der obere, der beweglichen Klemmbacke 6 zugewandte Endbereich der angeschrägten Schneide 17 der Klinge 12 stärker in den Zwischenraum 9 als deren unterer Endbereich.

Einen Schnitt durch das chirurgische Instrument 1 der Fig.7 entlang der Linie C-C zeigt die Fig.8. Dieser ist zu entnehmen, dass die Geometrie des Lagerbolzens 8 derjenigen der Darstellung aus der Fig.6 entspricht, der Lagerbolzen 8 also ohne den Zwischenabschnitt 21 mittleren Querschnitts ausgebildet ist. Die Lagerenden 19 des Lagerbolzens greifen hierbei mit ihren abgeflachten Bereichen 23 in an dem Schaft 4 vorgesehene Öffnungen 26, deren Ränder die Widerlager der abgeflachten Bereiche 23 bilden. Nicht dargestellt sind die die Befestigung des Lagerbolzens 8 an dem Schaft 4 sicherstellenden Nieten 13.

Die aus den Figuren 7 und 8 bekannte Offenstellung des chirurgischen Instruments ist erneut in der Darstellung der Fig.9 aus einer anderen Perspektive gezeigt, die in stirnseitiger Ansicht des distalen Endes des Instruments 1 die einander gegenüberliegenden Stirnflächen der Klemmbacken 5, 6 erkennen lässt. Gleichzeitig erkennt man daneben auch den Durchgriff der Trenneinrichtung 10 durch den Lagerbolzen 8, sowie das Gleiten der Trenneinrichtung in der in Fortsetzung der Durchtrittsöffnung 11 angeordneten, nutartigen Führung 14, die sich im wesentlichen vom proximalen bis zum distalen Ende der beiden Klemmbacken 5, 6 erstreckt. In den Schaft 4 hinein ragend ist die Fortsetzung der Klinge 12 der Trenneinrichtung 10 zu erkennen.

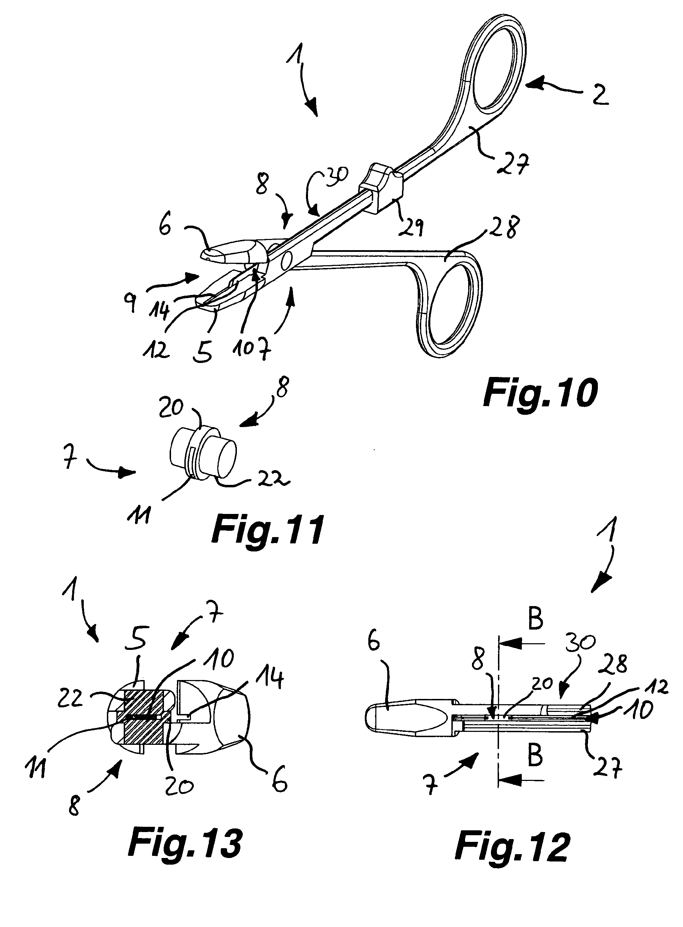

Die Fig.10 zeigt als weitere Ausführungsform ein chirurgisches Instrument 1, welches als Klemme für die offene Chirurgie ausgeführt ist. Die Klemme ist aus zwei Klemmenschenkeln 27, 28 aufgebaut, die an ihren dem Betrachter abgewandten Enden ringartige Griffteile 2 zum Eingriff je eines Fingers eines Benutzers aufweisen. Die Klemmenschenkel 27, 28 sind um ihre gemeinsame Lagerung 7 gegeneinander verschwenkbar, so dass die an den jeweiligen distalen Enden der Klemmenschenkel 27, 28 befindlichen Klemmbacken 5, 6 aus einer dargestellten Offenstellung in eine Schließstellung überführt werden können.

Die erwähnte Lagerung 7 ist im wesentlichen durch den in der Fig.11 einzeln gezeigten Lagerbolzen 8 gebildet, der einen Mittelabschnitt 20 größeren Querschnitts aufweist, in welchem sich eine Durchtrittsöffnung 11 zum Durchstecken der Trenneinrichtung 10 befindet, wobei sich an den Mittelabschnitt 20 zu beiden Seiten direkt die Endabschnitte 22 anschließen. An den Endabschnitten 22 sind die Klemmenschenkel 27, 28 gelagert. Die Trenneinrichtung 10 der Fig.10 besteht aus einer an dem einen Klemmenschenkel 27 angeordneten Klinge 12, die in den Klemmbacken 5, 6 durch die nutartige Führung 14 geführt ist. Die Trenneinrichtung 10 ist durch eine an dem betreffenden Klemmenschenkel 27 angeordnete und geführte Betätigungseinrichtung 29 in Art eines Schiebers in den zwischen den Klemmbacken 5, 6 befindlichen Zwischenraum vor- und zurückziehbar. Außerdem ist an dem Klemmenschenkel 27 eine die Klinge führende Halterung 30 vorgesehen.

Die Fig.12 zeigt eine Draufsicht auf den distalen Bereich des Instruments 1 mit den Klemmenschenkeln 27, 28 der Fig.10 und mit den den jeweiligen Klemmenschenkeln zugeordneten Klemmbacken 5, 6. Weiter ist in der Fig.12 zwischen den Klemmenschenkeln 27, 28 die Klinge 12 der Trenneinrichtung 10 sowie der Mittelabschnitt 20 des Lagerbolzens 8 erkennbar, der von der Trenneinrichtung 10 an der Durchtrittsöffnung 11 durchgriffen ist. Die die Klinge 12 der Trenneinrichtung 10 an der Klemmbacke 27 haltende und führende Halterung 30 ist nicht weiter dargestellt. Die beiden Endabschnitte 22 des Lagerbolzens greifen in der Darstellung der Fig.12 in die dafür vorgesehenen Ausnehmungen an den Klemmensschenkeln 27, 28 ein, so dass dort lediglich der Mittelabschnitt 20 mit vergrößertem Querschnitt, an welchem dich die von der Trenneinrichtung 10 durchgriffenen Durchtrittsöffnung 11 befindet, sichtbar ist.

Zu erkennen sind die in der Fig.12 nicht sichtbaren Endabschnitte 22 des Lagerbolzens 8 jedoch in der Fig.13, die einen Schnitt der Darstellung der Fig.12 entlang der Linie B-B zeigt. Die Klinge 12 der Trenneinrichtung 10 ist durch die Durchtrittsöffnung 11 gesteckt und die Klemmbacken 5, 6 sind an den Endabschnitten 22 des Lagerbolzens 8 gelagert, durch die Offenstellung der Klemmbacken 5, 6 ist erneut die nutartige Führung 14 an der einen Klemmbacke 6 erkennbar.

Die vorstehend beschriebene Erfindung betrifft demnach ein chirurgisches Instrument 1 mit zwei an einem Ende eines Schafts 4 oder eines Griffteils 2 befindlichen Klemmbacken 5, 6 mit einer einen durchgehenden Lagerbolzen 8 aufweisenden Lagerung 7, an welcher zumindest eine der Klemmbacken 5, 6 zwischen einer Offen- und einer Schließstellung schwenkbar gelagert ist, und mit einer im wesentlichen parallel zu der Längsachse des Instruments 1 in einen zwischen den Klemmbacken 5, 6 befindlichen Zwischenraum 9 verschiebbaren Trenneinrichtung 10. Um beispielsweise einen koagulierten Gewebeabschnitt sicher durchtrennen zu können weist der Lagerbolzen 8 eine sich entlang der Bewegungsrichtung der Trenneinrichtung 10 erstreckende Durchtrittsöffnung 11 auf, durch welche die Trenneinrichtung 10 durchsteckbar ist, wobei die größte Erstreckung der Durchtrittsöffnung 11 quer zur Bewegungsrichtung der Trenneinrichtung 10 wenigstens der Abmessung der Trenneinrichtung 10 in dieser Richtung entspricht.

## Patentansprüche

1. Chirurgisches Instrument mit zwei an einem Ende eines Schafts oder eines Griffteils befindlichen Klemmbacken, und mit einer einen durchgehenden Lagerbolzen aufweisenden Lagerung, an welcher zumindest eine der Klemmbacken zwischen einer Offen- und einer Schließstellung schwenkbar gelagert ist, und mit einer im wesentlichen parallel zu der Längsachse des Instruments in einen zwischen den Klemmbacken befindlichen Zwischenraum verschiebbaren Trenneinrichtung, **dadurch gekennzeichnet, dass** der durchgehende Lagerbolzen (8) eine sich entlang der Bewegungsrichtung der Trenneinrichtung (10) erstreckende Durchtrittsöffnung (11) aufweist, durch welche die Trenneinrichtung (10) durchsteckbar ist, und dass die größte Erstreckung der Durchtrittsöffnung (11) quer zur Bewegungsrichtung der Trenneinrichtung (10) wenigstens der Abmessung der Trenneinrichtung (10) in dieser Richtung entspricht und dass die Durchtrittsöffnung (11) für die Trenneinrichtung (10) quer zur Längsachse der Lagerung (7) schlitzartig verlaufend vorgesehen ist, wobei die größere Ausdehnung des die Durchtrittsöffnung (11) bildenden Schlitzes quer zur Längserstreckung des Lagerbolzens (8) verläuft.

2. Chirurgisches Instrument nach Anspruch 1, **dadurch gekennzeichnet, dass** die Trenneinrichtung (10) als Trenneinrichtung mit wenigstens einer Klinge (12) ausgebildet ist.

3. Chirurgisches Instrument nach Anspruch 1 oder 2, **dadurch gekennzeichnet, dass** der Lagerbolzen (8) als rotationssymmetrischer, insbesondere bereichsweise zylindrischer Körper mit gegebenenfalls sich änderndem Querschnitt ausgebildet ist.

4. Chirurgisches Instrument nach einem der Ansprüche 1 bis 3, **dadurch gekennzeichnet, dass** der Lagerbolzen (8) seinen größten Querschnitt quer zur Bewegungsrichtung der Trenneinrichtung (10) in einem Abschnitt aufweist, in welchem die Durchtrittsöffnung (11) angeordnet ist.

5. Chirurgisches Instrument nach einem der Ansprüche 1 bis 4, **dadurch gekennzeichnet, dass** die an der Lagerung (7) befindliche Durchtrittsöffnung (11) allseitig geschlossen ist.

6. Chirurgisches Instrument nach einem der Ansprüche 1 bis 4, **dadurch gekennzeichnet, dass** die an der Lagerung (7) befindliche Durchtrittsöffnung (11) mit einer insbesondere über die gesamte Längserstreckung der Durchtrittsöffnung (11) unterbrochenen Umfangswand einseitig offen ausgebildet ist.

7. Chirurgisches Instrument nach einem der vorhergehenden Ansprüche **dadurch gekennzeichnet, dass** die Durchtrittsöffnung (11) derart in der Mitte des Lagerbolzens (8) der Lagerung (7) angeordnet ist, dass deren Mitte eine Symmetrieebene hinsichtlich der sich zu beiden Seiten ersteckenden Teile des Lagerbolzens (8) bildet.

8. Chirurgisches Instrument nach einem der vorhergehenden Ansprüche, **dadurch gekennzeichnet, dass** ein mit der Durchtrittsöffnung (11) versehener Mittelabschnitt (20) des Lagerbolzens (8) einen größeren Querschnitt als dessen Lagerenden (19) aufweist.

9. Chirurgisches Instrument nach einem der vorhergehenden Ansprüche, **dadurch gekennzeichnet, dass** an dem Lagerbolzen (8) zwischen dem Mittelabschnitt (20) und dem jeweiligen Lagerende (19) ein Zwischenabschnitt (21) vorgesehen ist, dessen Durchmesser zwischen dem des Mittelabschnitts (20) und dem des Lagerendes (19) liegt.

10. Chirurgisches Instrument nach Anspruch 9, **dadurch gekennzeichnet, dass** die radiale Ausdehnung des Zwischenabschnitts (21) gleich groß oder größer als die größte radiale Ausdehnung des Durchtrittsöffnung (11) ist.

11. Chirurgisches Instrument nach einem der vorhergehenden Ansprüche, **dadurch gekennzeichnet, dass** die Ränder (24) der Eintrittsöffnung und/oder der Austrittsöffnung (25) der Durchtrittsöffnung (11) zumindest bereichsweise abgeflacht sind, insbesondere gegenüberliegende Längsränder (24) einer schlitzartigen Durchtrittöffnung (11) angefast sind.

12. Chirurgisches Instrument nach einem der vorhergehenden Ansprüche, **dadurch gekennzeichnet, dass** an den Lagerenden (19) des Lagerbolzens (8) Bereiche (23) zur Anlage eines Widerlagers vorgesehen sind, die die Rotationsymmetrie des Lagerbolzens (8) dort gegebenenfalls brechen.

13. Chirurgisches Instrument nach einem der vorhergehenden Ansprüche, **dadurch gekennzeichnet, dass** die Trenneinrichtung (10) mit mindestens einer Betätigungseinrichtung (3, 29) und gegebenenfalls mit einer Halterung (30) versehen ist, an welcher eine Klinge (12) der Trenneinrichtung (10) lösbar befestigt ist.

14. Chirurgisches Instrument nach einem der vorhergehenden Ansprüche, **dadurch gekennzeichnet, dass** die Trenneinrichtung (10) von dem proximalen bis in den Bereich des distalen Endes der Klemmbacken (5, 6) in einer in Verlängerung der Durchtrittsöffnung (11) an den Klemmbacken (5, 6) angeordneten, mit dieser fluchtenden Führung (14), insbesondere in einer Nut, geführt ist.

15. Koagulationsinstrument, insbesondere Hochfrequenz-Koagulationsinstrument mit einem chirurgischen Instrument (1) nach einem der vorhergehenden Ansprüche.

## Claims

1. A surgical instrument comprising two clamping jaws located at an end of a shaft or a grip part, and comprising a bearing having a continuous bearing pin, on which at least one of the clamping jaws is mounted so as to be able to pivot between an open position and a closed position, and comprising a separating means that can be displaced substantially parallel to the longitudinal axis of the instrument in a gap between the clamping jaws, **characterised in that** the continuous bearing pin (8) has a through-opening (11) extending along the direction of movement of the separating means (10), through which opening the separating means (10) can be passed, and **in that** the greatest extent of the through-opening (11) transverse to the direction of movement of the separating means (10) corresponds at least to the dimensioning of the separating means (10) in this direction, and **in that** the through-opening (11) for the separating means (10) is provided running in a slot-like manner transversely to the longitudinal axis of the bearing (7), wherein the greater extent of the slot forming the through-opening (11) runs transversely to the longitudinal extent of the bearing pin (8).

2. The surgical instrument according to Claim 1, **characterised in that** the separating means (10) is formed as a separating means having at least one blade (12).

3. The surgical instrument according to Claim 1 or 2, **characterised in that** the bearing pin (8) is formed as a rotationally symmetrical body, in particular that is cylindrical in areas, possibly having a changing cross section.

4. The surgical instrument according to one of Claims 1 to 3, **characterised in that** the bearing pin (8) has its greatest cross section transverse to the direction of movement of the separation device (10) in a portion in which the through-opening (11) is arranged.

5. The surgical instrument according to one of Claims 1 to 4, **characterised in that** the through-opening (11) located on the bearing (7) is closed on all sides.

6. The surgical instrument according to one of Claims 1 to 4, **characterised in that** the through-opening (11) located on the bearing (7) is formed with a peripheral wall that is open on one side and that in particular is interrupted over the total longitudinal extent of the through-opening (11).

7. The surgical instrument according to one of the preceding claims, **characterised in that** the through-opening (11) is arranged in the middle of the bearing pin (8) of the bearing (7), and **in that** the middle of said through-opening forms a plane of symmetry with respect to the parts of the bearing pin (8) extending on either side.

8. The surgical instrument according to one of the preceding claims, **characterised in that** a middle portion (20) of the bearing pin (8) provided with the through-opening (11) has a larger cross section than the bearing ends (19) thereof.

9. The surgical instrument according to one of the preceding claims, **characterised in that** an intermediate portion (21) is provided on the bearing pin (8) between the middle portion (20) and the respective bearing end (19), the diameter of said intermediate portion lying between that of the middle portion (20) and that of the bearing end (19).

10. The surgical instrument according to Claim 9, **characterised in that** the radial extent of the intermediate portion (21) is the same size as, or larger than the greatest radial extension of the through-opening (11).

11. The surgical instrument according to one of the preceding claims, **characterised in that** the edges (24) of the inlet opening and/or of the outlet opening (25) of the through-opening (11) are flattened at least in areas, in particular opposite longitudinal edges (24) is a slot-like through-opening (11) are chamfered.

12. The surgical instrument according to one of the preceding claims, **characterised in that** areas (23) for the attachment of an abutment are provided at the bearing ends (19) of the bearing pin (8) and may break there the rotational symmetry of the bearing pin (8).

13. The surgical instrument according to one of the preceding claims, **characterised in that** the separating means (10) is provided with at least one actuation means (3, 29) and optionally with a mount (30), to which a blade (12) of the separating means (10) is detachably secured.

14. The surgical instrument according to one of the preceding claims, **characterised in that** the separating means (10) is guided from the proximal end of the clamping jaws (5, 6) into the region of the distal end of said clamping jaws in a guide (14), in particular in a groove, that is arranged in the extension of the through-opening (11) on the clamping jaws (5, 6), aligned therewith.

15. A coagulation instrument, in particular a highfrequency coagulation instrument, comprising a surgical instrument (1) according to one of the preceding claims.

## Revendications

1. Instrument chirurgical comportant deux mâchoires de serrage se trouvant à une extrémité d'une tige ou d'une pièce de préhension et un palier présentant un boulon d'appui continu sur lequel au moins une des mâchoires de serrage s'appuie en pivotement entre une position ouverte et une position fermée et un dispositif de sectionnement déplaçable sensiblement à la parallèle de l'axe longitudinal de l'instrument dans un espace se trouvant entre les mâchoires de serrage, **caractérisé en ce que** le boulon d'appui continu (8) présente un orifice de passage (11) s'étendant le long du sens de mouvement du dispositif de sectionnement (10) et dans lequel le dispositif de sectionnement (10) peut être fiché, et que l'extension maximale de l'orifice de passage (11) transversalement au sens de mouvement du dispositif de sectionnement (10) équivaut au moins à la dimension du dispositif de sectionnement (10) dans ce sens et que l'orifice de passage (11) pour le du dispositif de sectionnement (10) est réalisé sous forme d'une fente et orienté transversalement à l'axe longitudinal du palier (7), l'extension maximale de la fente constituant l'orifice de passage (11) étant orientée transversalement à l'extension longitudinale du boulon d'appui (8).

2. Instrument chirurgical selon la revendication 1, **caractérisé en ce que** le dispositif de sectionnement (10) se présente sous forme d'un dispositif de sectionnement doté d'au moins une lame (12).

3. Instrument chirurgical selon la revendication 1 ou 2, **caractérisé en ce que** le boulon d'appui (8) se présente sous forme d'un corps cylindrique symétrique en rotation, en particulier symétrique par endroits, ayant une section transversale éventuellement variable.

4. Instrument chirurgical selon une des revendications 1 à 3, **caractérisé en ce que** le boulon d'appui (8) a sa section transversale maximale transversalement au sens de mouvement du dispositif de sectionnement (10) dans une section dans laquelle l'orifice de passage (11) est pratiqué.

5. Instrument chirurgical selon une des revendications 1 à 4, **caractérisé en ce que** l'orifice de passage (11) se trouvant au niveau du palier (7) est fermé de tous côtés.

6. Instrument chirurgical selon une des revendications 1 à 4, **caractérisé en ce que** l'orifice de passage (11) se trouvant au niveau du palier (7) a une conformation ouverte d'un côté avec une paroi circonférentielle interrompue en particulier sur toute l'extension longitudinale de l'orifice de passage (11)..

7. Instrument chirurgical selon une des revendications précédentes, **caractérisé en ce que** l'orifice de passage (11) est pratiqué au centre du boulon d'appui (8) du palier (7), de sorte que son centre forme un plan de symétrie par rapport aux parties s'étendant vers les deux côtés du boulon d'appui (8).

8. Instrument chirurgical selon une des revendications précédentes, **caractérisé en ce qu'**une section centrale (20) pourvue de l'orifice de passage (11) du boulon d'appui (8) a une section transversale supérieure à celle de ses extrémités d'appui (19).

9. Instrument chirurgical selon une des revendications précédentes, **caractérisé en ce qu'**il est prévu sur le boulon d'appui (8), entre la section centrale (20) et l'extrémité d'appui respective (19), une section intermédiaire (21) dont le diamètre se situe entre celui de la section centrale (20) et celui de l'extrémité d'appui (19).

10. Instrument chirurgical selon la revendication 9, **caractérisé en ce que** l'extension radiale de la section intermédiaire (21) est supérieure ou égale à l'extension radiale maximale de l'orifice de passage (11).

11. Instrument chirurgical selon une des revendications précédentes, **caractérisé en ce que** les bords (24) de l'orifice d'entrée et/ou de l'orifice de sortie (25) de l'orifice de passage (11) sont aplatis du moins par endroits, en particulier les bords longitudinaux opposés (24) d'un orifice de passage en forme de fente (11).

12. Instrument chirurgical selon une des revendications précédentes, **caractérisé en ce que**, aux extrémités d'appui (19) du boulon d'appui (8), sont prévues des zones (23) d'appui d'un contre-palier qui y rompent éventuellement la symétrie de rotation du boulon d'appui (8).

13. Instrument chirurgical selon une des revendications précédentes, **caractérisé en ce que** le dispositif de sectionnement (10) est équipé d'au moins un dispositif d'actionnement (3, 29) et éventuellement d'un support (30) auquel une lame (12) du dispositif de sectionnement (10) est fixée de manière amovible.

14. Instrument chirurgical selon une des revendications précédentes, **caractérisé en ce que** le dispositif de sectionnement (10) est guidé de l'extrémité proximale jusqu'au niveau de l'extrémité distale des mâchoires de serrage (5, 6) dans un guide (14) disposé dans le prolongement de l'orifice de passage (11) sur les mâchoires de serrage (5, 6) et aligné avec celui-ci.

15. Instrument de coagulation, en particulier instrument de coagulation à haute fréquence, comportant un instrument chirurgical (1) selon une des revendications précédentes.
